# EUROPEAN PATENT SPECIFICATION

(11) **EP 4 543 974 B1**
(45) Date of publication and mention of the grant of the patent: **09.12.2026**
(21) Application number: 23735302.4
(22) Date of filing: 26.06.2023
(51) Int. Cl.: C08J 11/14, C07C 51/06, C07C 55/02, C07C 209/50, C07C 209/62, C07C 211/09, C08G 69/48

(54) **METHOD FOR RECYCLING A POLYAMIDE UNDER ACIDIC CONDITIONS**
VERFAHREN ZUR WIEDERVERWERTUNG VON POLYAMID UNTER SAUREN BEDINGUNGEN
PROCÉDÉ DE RECYCLAGE D'UN POLYAMIDE DANS DES CONDITIONS ACIDES

(30) Priority: 26.06.2022 EP 22315129
(43) Date of publication of application: 30.04.2025
(73) Proprietor: Syensqo Specialty Polymers USA, LLC, Alpharetta, GA 30005-3914 (US)
(72) Inventor: TOURAUD, Franck, 69190 Saint-Fons (FR); SERVEL, Clement, 69190 Saint-Fons (FR); JEOL, Stéphane, 69190 Saint-Fons (FR)
(74) Representative: Senninger, Thierry
(86) International application number: PCT/EP2023/067332
(87) International publication number: WO 2024/002975

(56) References cited:
- US-A- 2 407 896
- US-A- 3 069 465

## Description

The present disclosure relates to an environmentally respectful method for recycling a polyamide into its constitutive monomers under acidic conditions. The method involves depolymerizing the polyamide into its monomers and recovering them.

### [Context of the invention]

Plastics are inexpensive and durable materials, which can be used to manufacture a variety of products that find use in a wide range of applications, so that the production of plastics has increased dramatically over the years since their discovery. It is estimated that about 40% of these plastics are used for single-use disposable applications, such as packaging, agricultural films, disposable consumer items or for short-lived products that are discarded within a year of manufacture. Because of the durability of the polymers involved, substantial quantities of plastics are piling up in landfill sites and in natural habitats worldwide, generating increasing environmental problems. Even degradable and biodegradable plastics may persist for decades depending on local environmental factors, like levels of ultraviolet light exposure, temperature, presence of suitable microorganisms, etc.

One solution to reduce environmental and economic impacts correlated to the accumulation of plastic is closed-loop recycling wherein plastic material is mechanically reprocessed to manufacture new products. For example, one of the most common closed-loop recycling is the polyethylene terephthalate (PET) recycling. PET wastes are subjected to successive treatments leading to a food-contact-approved recycled PET, which is collected, sorted, pressed into bales, crushed, washed, chopped into flakes, melted and extruded in pellets and offered for sale. Then, these recycled PET may be used to create fabrics for the clothing industry or new packaging such as bottles or blister packs, etc.

However, plastic wastes are generally collected all together, so that plastic bales contain a mixture of different plastics, the composition of which may vary from source to source, and the proportions of which may vary from bale to bale. Consequently, recycling processes require preliminary selection to sort out the plastic products according to their composition, size, resin type, colour, functional additives used, etc.

Another potential process for recycling plastics consists of chemical recycling allowing recovering the monomers of the polymer. The resulting monomers may then be used to re-manufacture plastic materials (the same or other plastic materials) or to make other synthetic chemicals. While this chemical and enzymatic depolymerization process has been well optimized over the years for PET with very high yield of recycled ethylene glycol and recycled terephthalic acid, there is still need to develop similarly an efficient and optimized process for polyamides.

### [Background art]

EP 0737666 discloses a process for the depolymerization of one or more polyamides in the presence of at least a nitrogen-containing compound. The nitrogen-containing compound can be NH₃, a (cyclo)aliphatic amine, a (cyclo)aliphatic diamine, a (cyclo)aliphatic polyamine, an aromatic diamine and/or an aromatic polyamine. The hydrolysis is not performed in acidic conditions.

US 6,087,494 discloses a process for depolymerization of one or more polyamides into monomeric units, comprising depolymerizing said one or more polyamides at a pressure of between about 0.2 and about 20 MPa in the presence of at least one alkali metal compound, at least one alkaline-earth metal compound or a mixture thereof. The temperature is higher than 150°C.

ACS Sustainable Chem. Eng. 2020, 8, 16274-16282 discloses the treatment of aliphatic PAs (PA 6,6, PA 10,10, PA 11, and PA 12) into their constituent monomers through hydrolysis under microwave irradiation using hydrochloric acid as an acid catalyst. The temperature applied is higher than 150°C. Moreover, the use of microwave induces challenges for the industrialization of the method disclosed. There is also no exact mention of the proportions of water used in the experiments.

WO 9700846 discloses the hydrolysis of polyamide with nitric acid.

GB 1272302 discloses the hydrolysis in acidic conditions of a polyamino acid such as polyamide 6 in the presence of an alcohol.

US 6,020,486 discloses the hydrolysis of a polyamide with an acid in the presence of an alcohol. The temperature used is higher than 150°C.

US 3,069,465 (D2) discloses a process for recovering adipic acid and hexamethylenediamine from PA66 comprising the steps of continuously hydrolyzing the polyamide in aqueous sulfuric acid of from about 30% to about 70% concentration, at a temperature of from 75-140°C. The process involves a neutralising step with Ca(OH)₂ to release the hexamethylenediamine. The proportion of polyamide (PA) in the example is only 11 wt.% and the ratio H/N is above 7.0.

US 2,407,896 (D1) discloses the acidic hydrolysis of PA66 with the following proportions PA66:water:H₂SO₄ 1:1:1 (33.3% PA; 33.3% water; acid 33.3%) and is based on the use of Ca(OH)₂ to release the hexamethylenediamine. It is disclosed in column 3 - lines 29-34 that the method is also based on several steps of hydrolysis and recovery of the adipic acid. D1 discloses in particular in column 3 - lines 18-21 that the process proceeds satisfactorily only if the adipic acid is removed from the hydrolysis mixture a plurality of times.

DE 69309709 describes the hydrolysis and oxidation of polyamides. PA6, PA66 and PA12 are mentioned as possible polyamides. The reaction is based on the fact that nitroso groups are dissolved in the hydrolysis medium. There is no nitroso compounds added in the method of the present invention.

WO 2022/058291 discloses an improved process for the acid hydrolysis of polylaurolactam with sulfuric acid at a temperature between 125 and 190°C, preferably at a temperature higher than 160°C. The weight ratio of H₂SO₄ / polyamide used is preferably 1:0.1 to 1:1.

### [Technical problem to be solved]

There is a need for an efficient and environmentally-safe method of recovery of the monomers from a polyamide that can achieve a high degree of hydrolysis of the polyamide into its constituting monomers. The method should also preferably release a reduced amount of salt into the environment.

The method should also be easily scalable and be implemented under conditions that are not too harsch for the equipment used (therefore also limiting the corrosion of equipment).

The method of the invention aims at solving these technical problems.

### [Brief disclosure of the invention]

The invention is disclosed in the appended claims.

The method of the invention is disclosed in one of the claims 1-14.

More precisions and details are now provided below.

### [General definitions]

wt.% is a percentage by weight.

When numerical ranges are indicated, range ends are included.

### [Disclosure of the invention]

The method of recovery of the monomers from a polyamide (PA) of the AABB type is defined in claim 1 and comprises the following steps:
- a) a product (P) comprising the polyamide (PA) is optionally comminuted;
- b) an aqueous medium comprising water, the product (P) and an acid (Ac) is heated to induce the dissolution and the hydrolysis of the polyamide (PA);
- c) a stream (S) comprising the diacid (A) and the diamine (B) in the form of a salt with the acid (Ac) which is recovered at the end of step b) is further processed in order to either separate and recover the diacid (A) and the diamine (B) (embodiment E1) or collect the salt formed by the diacid (A) and the diamine (B) (embodiment E2);

the polyamide (PA) of the AABB type being notably prepared by polycondensation of:
   - at least one diacid (A) selected in the group of C₃-C₁₈ aliphatic diacids, isophthalic acid and terephthalic acid; and
   - at least one diamine (B) selected in the group consisting of C₂-C₁₈ aliphatic diamines, C₄-C₁₈ cycloaliphatic diamines and diamines of formula (IV): 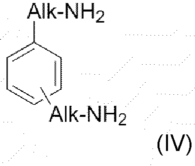 wherein Alk is a C₁-C₆ linear or branched alkylene group;
wherein step b) is performed with the following conditions:
   (i) the initial proportions in the aqueous medium are the following:
      - proportion of polyamide (PA): at least 15.0 wt.%;
      - proportion of water: between 20.0 and 70.0 wt.%;
      - the remainder as acid (Ac) with the condition that the proportion of acid (Ac) is at least 2.0 wt.%, preferably at least 2.5 wt.%;
      these proportions being based on the total weight of polyamide (PA), water and acid (Ac);
      - the molar ratio (H / N) representing the amount of H from the acid (Ac) over the amount of N from the amide bonds of the polyamide (PA) between 1.1 and 7.0;
   (ii) the temperature is between 80°C and 150°C;
wherein after step b), the method comprises a total or partial removal of the unreacted acid (Ac) from the reaction mixture;
wherein:
   - according to embodiment (E1), step c) comprises a separation substep c1), wherein the diacid (A) and the salt of the diamine (B) are separated and a neutralisation substep c2), wherein the salt of diamine (B) separated from the diacid (A) is neutralised with at least one inorganic base of formula XOH, X being Li, Na, K or a combination of two or more of these cations to release the diamine (B);
   - according to embodiment (E2), step c) comprises a neutralisation substep c2) in which the salt of diamine (B) is neutralised with an inorganic base of formula XOH to release the diamine (B);
and wherein the α ratio is less than or equal to 2.0, this ratio being defined as the molar ratio = [salt of the acid (Ac) with XOH released by the method] / [amide bonds of the polyamide (PA)].

### Product (P)

The product (P) comprises at least one polyamide (PA) of the AABB type. An AABB type polyamide is a polyamide resulting from the polycondensation of a diacid and a diamine.

The polyamide (PA) may notably be prepared by polycondensation of:
- at least one diacid (A) selected in the group of C₃-C₁₈ aliphatic diacids, isophthalic acid and terephthalic acid; and
- at least one diamine (B) selected in the group consisting of C₂-C₁₈ aliphatic diamines, C₄-C₁₈ cycloaliphatic diamines and C₈-C₁₈ arylaliphatic diamines.

The diacid (A) may be an aliphatic diacid represented by general formula (I) HOOC-Alk-COOH, wherein Alk is a C₂-C₁₈ linear or branched alkylene group. For instance, the aliphatic diacid (A) may be adipic acid or sebacic acid.

The diamine (B) may be an aliphatic diamine represented by the general formula (II) H₂N-Alk-NH₂, wherein Alk is a C₂-C₁₈ linear or branched alkylene group. For instance, the aliphatic diamine may be hexamethylene diamine, 2,2,4-trimethyl-1,6-hexanediamine, 2,4,4-trimethyl-1,6-hexanediamine, 1,3-diaminopentane, 1,5-diaminohexane or 1,8-diamino-nonane.

The diamine (B) may also be a cycloaliphatic diamine. A cycloaliphatic diamine is a diamine comprising at least one cycloaliphatic group between the two NH₂. The cycloaliphatic diamine may more particularly be selected in group consisting of isophorone diamine, norbornane diamine, 1,3-bis(aminomethyl)cyclohexane (1,3-BAC), 1,4-bis(aminomethyl)cyclohexane (1,4-BAC) and the diamines of formula (III): 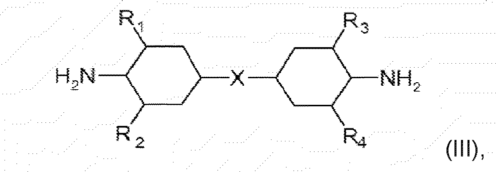 wherein R₁, R₂, R₃ and R₄ are independently selected in the group of H and C₁-C₆ alkyl groups and X is a C₁-C₁₀ alkylene groups. In formula (III), X is more particularly a methylene group. In formula (III), R₁, R₂, R₃ and R₄ are more particularly independently selected in the group consisting of H and CH₃.

The cycloaliphatic diamine may more particularly be selected in group consisting of isophorone diamine, norbornane diamine, 1,3-BAC, 1,4-BAC, para-bis(aminocyclohexyl)-methane (PACM) and bis-(3-methyl-4-aminocyclohexyl)-methane (MACM).

The diamine (B) may be a diamine of formula (IV): 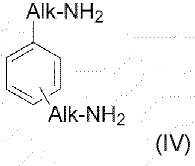 wherein Alk is a C₁-C₆ linear or branched alkylene group. The diamine of formula (IV) may be for instance m-xylylene diamine (MXDA) or p-xylylene diamine (PXDA).

The polyamide (PA) may more particularly be selected in the group consisting of polyamide 6.6, polyamide 6.9, polyamide 6.10, polyamide 6.12, polyamide 10.10, polyamide 4.6, polyamide 4.9, polyamide 4.10, polyamide 12.12, polyamide 10.12, polyamide MXD6, polyamide MXD6/PXD6, polyamide MXD6/MXDI, polyamide 6T/66, polyamide 6T/6I/66, PA 6T/6I and mixtures thereof *(note: MXD designates a structural unit with MXDA as the diamine; PXD designates a structural unit with PXDA* as *the diamine; I designates designates a structural unit with isophthalic acid as the diacid; T designates designates a structural unit with terephtahalic acid* as *the diacid).*

The polyamide (PA) may more particularly be polyamide 6.6, polyamide 6T/66, polyamide 6.10 or MXD6.

Product (P) may also comprise another polymer that is not a polyamide (PA) of the AABB type as defined above. This polymer may be either blended with the polyamide (PA) and/or physically present with polyamide (PA) in product (P) but not blended. Preferably, the other polymer is not a polyamide.

Product (P) usually also comprises at least one polymer additive (A). The polymer additive (A) may be selected in the group consisting of fillers, colorants, dyes, pigments, lubricants, plasticizers, flame retardants, nucleating agents, heat stabilizers, UV stabilizers, elastomers, adhesives, antioxidants and processing aids. The polymer additive (A) may more particularly be selected in the group consisting of fillers, colorants, dyes, pigments, lubricants, elastomers and heat stabilizers.

The proportion of polyamide (PA) in product (P) is generally at least 30.0 wt.%, more particularly at least 40.0 wt.%. This proportion may be at least 50.0 wt.% or even at least 60.0 wt.%. This proportion may be 100 wt.% if product (P) consists of polyamide (PA). Yet, this situation is rare as the method of the invention is meant to apply to products of our day-to-day life for which polymer additives are usually present in combination with the polyamide (PA). The proportion of polyamide (PA) in product (P) is generally less than 99.9 wt.%, more particularly less than 99.5 wt.%.

The number average molecular weight Mₙ of the polyamide (PA) is generally between 500 and 50,000 g/mol, preferentially between 1,000 and 35,000 g/mol, even more preferentially between 2,000 g/mol and 20,000 g/mol. The Mn is determined by the equation Mn=2,000,000/[EG], with [EG] being the concentration in meq/kg of the end-groups of the polyamide (PA). The usual end-groups in polyamides are -NH₂ and -COOH. Yet, those end-groups may in some processes be converted, partly or totally, into other end-groups by reaction with an end-capping agent. Examples of end-capping agent are monofunctional molecules containing an amine or a carboxylic acid such as acetic acid, benzoic acid, propionic acid.

### Optional step a)

Product (P) to be treated may be in various forms. Indeed, product (P) may for instance be in the form of pellets, powders, films, flakes, molded or extruded or 3D printed parts, tubes, filaments, yarns, textiles, fabrics or under any type of geometry.

More particularly, product (P) may be in the form of a film comprising at least one layer comprising or made of the polyamide (PA). Product (P) may more particularly be in the form of a multilayer film comprising at least one layer comprising or made of the polyamide (PA). For example, product (P) may be in the form of a multilayer film comprising a layer comprising or made of the polyamide (PA), notably MXD6, in between two layers comprising polyethylene terephthalate (PET).

Yet, the size of product (P) is preferably reduced to decrease the time of step b).

Product (P) is therefore preferably in the form of particles. The particles of product (P) preferably exhibit a size lower than 10.0 mm, preferably lower than 5.0 mm, even preferably lower than 3.0 mm.

Step b): the method of the invention advantageously does not comprise an additional hydrolysis step after the recovery of the diacid (A).

The method of D1 comprises a plurality of hydrolysis steps and a corresponding number of removals of the diacid whereas the method of the invention is preferably comprises a single hydrolysis step b).

In step b), an aqueous medium comprising water, the product (P) and an acid (Ac) is heated to induce the dissolution and the hydrolysis of the polyamide (PA). The dissolution may be total or partial.

The acid (Ac) has at least one pKa lower than 0 and is selected in the group consisting of HCl, HBr, methane sulfonic acid and combinations of two or more of these acids;.
All these acids exhibit at least one pKa lower than 0: HCl: -6.3; HBr: -8.7;; methane sulfonic acid: -1.9.

The method is applicable to combinations of two of the afore-mentioned acids. Yet, to manage industrially the flows of products, it is preferable to use only one acid (Ac).

The acid (Ac) is preferably HCl.

HNO₃ and other nitrogen-containing inorganic acids are not suitable as they tend to release NO₂ and are consequently harmful for the environment. Weak acids are also avoided. The method of the present invention therefore does not use any inorganic acid, HNO₃ and other nitrogen-containing inorganic acids. For the same reasons, the method of the present invention therefore does not need the addition of any nitroso compounds as in DE 69309709. The liquid medium thus preferably does not comprise any nitroso compounds.

### Conditions of step b)

(i) initial proportions in the aqueous medium: step b) is performed with the following initial proportions in the aqueous medium:
   - proportion of polyamide (PA): at least 15.0 wt.%;
   - proportion of water: between 20.0 and 70.0 wt.%;
   - the remainder as acid (Ac) with the condition that the proportion of acid (Ac) is at least 2.0 wt.%, preferably at least 2.5 wt.%.
   These proportions are expressed in wt.% and based on the total weight of polyamide (PA), water and acid (Ac) in the liquid medium.

The initial proportion of polyamide (PA) in the aqueous medium is at least 15.0 wt.%. This proportion may be between 15.0 wt.% and 55.0 wt.%, preferably between 15.0 wt.% and 35.0 wt.%, preferably between 15.0 wt.% and 30.0 wt.%. This proportion is calculated by taking into account the proportion of the polyamide (PA) in product (P). The higher the proportion of polyamide (PA) in the liquid medium, the better the productivity. Yet, the proportion is limited to take into account the increase over time of the viscosity of the liquid medium and the need to keep a sufficient quantity of acid (Ac) to maintain a suitable kinetics of depolymerization.

The proportion of water in the aqueous medium is between 20.0 wt.% and 70.0 wt.%. This proportion may be between 30.0 wt.% and 70.0 wt.% or between 35.0 wt.% and 65.0 wt.%. For clarity's sake, it is noted that the proportion of water used in the context of the invention, unless otherwise expressed, takes into account the water added and the water that may stem from the solution of the acid (Ac).

The initial proportion of the acid (Ac) corresponds to the complement to 100 wt.%. **In** other words, proportion of acid (Ac) in wt.% = 100% - proportion of PA in wt.% - proportion of water in wt.%. The minimal proportion of the acid (Ac) in the liquid medium is 2.0 wt.%, preferably at least 2.5 wt.%.

The initial proportion of the acid (Ac) is generally between 2.0 wt.% and 55.0 wt.%. This proportion may be between 5.0 wt.% and 55.0 wt.%.

For clarity's sake, it is noted that the proportion of the acid (Ac) in the liquid medium used in the context of the invention, unless otherwise expressed, is given as the proportion of the pure acid. For instance, a proportion of 20.0 wt.% refers to 20.0 wt.% of pure HCl irrespective of the strength of the solution (e.g. solution of HCl at 37 wt.%). For clarity's sake also, if the acid (Ac) corresponds to a combination of two or more of the acids as defined above, the proportions of acid (Ac) given herein correspond to the total proportions of the acids.

The initial molar ratio (H/N) representing the amount of H from the acid (Ac) over the amount of N from the amide bonds of the polyamide (PA) is between 1.1 and 7.0. This ratio is preferably at least 2.0. This ratio may preferably be between 2.5 and 7.0.

Specific conditions of the method are now provided:
- proportion of polyamide (PA): between 15.0 and 55.0 wt.%;
- proportion of water: between 20.0 and 70.0 wt.%;
- proportion of acid (Ac): between 10.0 and 55.0 wt.%;
- H/N between 2.0 and 7.0.

Specific other conditions of the method are now provided:
- proportion of polyamide (PA): between 15.0 and 35.0 wt.%;
- proportion of water: between 20.0 and 55.0 wt.%;
- proportion of acid (Ac): between 12.0 and 50.0 wt.%;
- H/N between 2.5 and 3.5.

Specific other conditions of the method are now provided:
- proportion of polyamide (PA): between 15.0 and 30.0 wt.%;
- proportion of water: between 30.0 and 45.0 wt.%;
- proportion of acid (Ac): between 12.0 and 50.0 wt.%;
- H/N between 2.5 and 3.5.

**In** addition to water, product (P) and acid (Ac), the aqueous medium may also comprise an organic component selected in the group of an alcohol, ketone and combination thereof. The organic component is typically liquid at ambiant temperature. The organic component preferably comprises less than 10 carbon atoms. The alcohol may more particularly be selected in the group consisting of methanol, ethanol, propanol, butanol and combination thereof. The ketone may more particularly be selected in the group consisting of acetone, propanone, butanone and combination thereof.

Step b) is performed in batch, in semi-continuous or in continuous. It is preferably performed in batch to control effectively the conditions and the proportions of components in the liquid medium. Moreover, working in batch also ensures an easier industrial implementation of the method.

(ii) temperature of step b): the temperature at which step b) is performed is between 80°C and 150°C, preferably between 100°C and 150°C, preferably between 100°C and 130°C.

The pressure in the tank in which step b) is performed is the natural pressure developed by the heated liquid medium. The pressure inside the tank is generally between 1.0 and 6.0 bar.

The duration of step b) depends on the operative conditions and the degree of hydrolysis R targeted. For a degree of hydrolysis R of at least 90.0%, the duration of step b) is generally between 1.0 hour and 72.0 hours, more particularly between 2.0 and 24.0 hours, even more particularly between 6.0 and 20.0 hours. The duration of the hydrolysis from a degree of hydrolysis of R=0 to R=90.0% is generally between 6.0 and 10.0 hours.

According to a preferable embodiment, step b) is performed without any microwave irradiation. This ensures an easier industrial implementation of the method.

**α ratio:** the method of the invention makes it possible to have a controlled release of the salt generated by the neutralisation with the base XOH which is needed to recover the diamine (B) in its free form. For instance, if the acid used in step b) is HCl and the base used is NaOH, the salt of the amine is in the form -NH₃⁺Cl⁻ and the neutralisation of the salt with NaOH releases NaCl as a salt.

The α ratio is defined as the molar ratio of the salt of the acid (Ac) with XOH that is released by the method / amide bonds in the polyamide (PA): $molar α ratio = \frac{salt of acid \left(Ac\right) with XOH which is released}{amide bonds in the polyamide \left(PA\right)}$

The α ratio is calculated for the overall method. The lower α, the better impact of the method on the environment. α is typically ≥ 1.0.

The method may release more than one salt. Indeed, as the method of the invention may use a combination of acids (Ac) and/or a combination of inorganic bases XOH, more than one salt can be released. For instance, if the acid (Ac) is HCl and the method uses a combination of NaOH and KOH in step c), the salts released are NaCl and KCI. The calculation of α ratio takes into account all the salts released by the neutralization.

As the acid is a volatile acid, α is less than or equal to 2.0 (≤ 2.0).

Removal of the acid (Ac): after step b), the method comprises a partial or total removal of the unreacted acid (Ac) from the reaction mixture. The removal is easier since the acid (Ac) is volatile. HCl, HBr or methanesulfonic acid are acids that can be easily removed from the aqueous medium, notably by distillation.

The acid (Ac) may be removed partially or totally from the reaction mixture by heating and/or applying vacuum to the reaction mixture.

The removal helps reduce the α ratio. α is thus be less than or equal to 2.0 (≤ 2.0). α is preferably less than or equal to 1.5 (≤ 1.5), preferably less than or equal to 1.4 (≤ 1.4), preferably less than or equal to 1.3 (≤ 1.3), preferably less than or equal to 1.2 (≤ 1.2). See example 14.

The removed acid (Ac) may be recycled to be reused in step b) of the method. Before being reused, the removed acid (Ac) may optionally be concentrated.

The removal of the acid (Ac) may be performed before substep c1) in embodiment (E1).

**Degree of hydrolysis R:** the degree of hydrolysis R achieved at the end of step b) is preferably at least 90.0 mol%, preferably at least 95.0 mol%. This ratio corresponds to the degree of conversion of the hydrolysis of the polyamide (PA). The degree of conversion is defined as the decrease in the quantity of a reactant divided by the initial quantity thereof (IUPAC definition).

The degree of hydrolysis R can be followed easily through analysis by ¹H NMR of samples of the reaction mixture collected over time. For instance, dried samples may be analysed by ¹H NMR in deuterated trifluoroacetic acid.

With the method of the present invention, it is possible to reach a degree of hydrolysis R of at least 90.0 mol%, or even at least 95.0 mol%, for a duration of step b) of less than 9.0 hours, preferably less than 8.0 hours.

Productivity ratio R*: the method of the invention makes it possible to have a high productivity ratio. This ratio is defined for degree of hydrolysis R of at least 90.0% as: initial proportion in wt.% of polyamide (PA) x R (%) / (initial proportion in wt.% of water + initial proportion in wt.% of acid) x 100. The higher this ratio, the more efficient is the method as it implies that for a given volume of the tank used for the hydrolysis, it is possible to occupy the volume of the tank with as much product (P) as possible.

The method of the invention makes it possible to have a ratio R* of at least 20.0%, preferably at least 25.0%, preferably at least 30.0%.

**Step c):** in step c), a stream (S) comprising the diacid (A) and the diamine (B) in the form of a salt with the acid (Ac) which is recovered at the end of step b) is further processed in order to either separate and recover the diacid (A) and the diamine (B) (embodiment E1) or collect the salt formed by the diacid (A) and the diamine (B) (embodiment E2).

It is preferable to have a stream (S) substantially free of the solid materials present in the aqueous medium which is obtained at the end of step b). These solid materials are generally the additives that may be present in product (P) (e.g. the fillers) and/or the particles of the polymer which have not been dissolved and depolymerized and/or the particles of the other polymer(s) that may be present in product (P). Filtration of stream (S) is a usual and convenient way to perform the separation of the solid materials.

Embodiment (E1): the stream (S) comprising the diacid (A) and the diamine (B) in the form of a salt with the acid (Ac) which is recovered at the end of step b) is further processed in order to either separate and recover the diacid (A) and the diamine (B).

Embodiment (E1) is conveniently based on crystallization and distillation.

Embodiment (E1) comprises at least one crystallisation step and/or at least one distillation step.

According to this embodiment, step c) comprises a separation substep c1) and a neutralisation substep c2). In substep c1), the diacid (A) and the salt of the diamine (B) are separated; and then in substep c2), the salt of diamine (B) separated from the diacid (A) is neutralised with an inorganic base of formula XOH, X being Li, Na, K or a combination of two or more of these cations to release the diamine (B). X is preferably NaOH.

The separation of the diacid (A) and the salt of the diamine (B) may be performed by crystallisation. Crystallisation is a separation technique that makes use of differences in solubility of the components that are present in the solution. For instance, in the case of PA 66, a substantial part of the diacid (A) (adipic acid) can crystallise at ambient temperature while the salt of the diamine (B) (hexamethylene diamine) can be left in the aqueous medium. If the aqueous medium is cooled below ambient temperature, more adipic acid crystallizes which helps increase the yield of recovery of the adipic acid.

After neutralisation with the inorganic base XOH, the diamine (B) is released and can be recovered, for instance by distillation.

Both the diacid (A) and the diamine (B) recovered can be further purified to the level of purity requested by the final end-use.

Step c) is applicable to a polyamide (PA) based on one diacid (A) and one diamine (B), such as PA66, PA6.10 or MXD6. The diacid (A) and the salt of the diamine (B) can be separated by crystallisation.

Step c) is applicable to a polyamide (PA) based on at least two diacids (A) and one diamine (B). The diacids (A) and the salt of the diamine (B) can be separated by crystallisation. The diacids (A) can be further separated from one another by other crystallization steps.

Step c) is applicable to a polyamide (PA) based on one diacid (A) and at least two diamines (B). The diacid (A) and the salts of the diamines (B) can be separated by crystallisation. After neutralization by XOH, distillation may be used to further separate the diamines (B) from one another.

**Embodiment (E2):** the stream (S) comprising the diacid (A) and the diamine (B) in the form of a salt with the acid (Ac) which is recovered at the end of step b) is further processed in order to collect the salt formed by the diacid (A) and the diamine (B). Embodiment (E2) then comprises a substep c2) in which the salt of diamine (B) is neutralised with an inorganic base of formula XOH to release the diamine (B).

The salt formed by the reaction of the diacid (A) and the diamine (B) may be recovered and optionally purified, e.g. by crystallisation. The salt may be engaged again in a polycondensation.

Embodiment (E1) is preferred as it makes it possible to recover the two monomers which can be used in a polycondensation or can be commercialised separately.

### [Experimental section]

### Example 1: hydrolysis of PA66 with HCl

A 300 ml glass reactor equipped with a reflux condenser and a mechanical stirrer is loaded with 30.0 g of recycled pellets (3-5 mm) of unfilled polyamide 66 (100% of PA, 0.266 mol of amide bonds) and 78.4 g of hydrochloric acid 37 wt.% aqueous solution (0.795 mol). The reaction mixture is immersed in an oil bath at 120°C and kept at reflux for 10 hours.

Full dissolution of PA66 granules is observed after 45 minutes (see Table I).

Samples are taken regularly and dried in a vacuum oven overnight. The hydrolysis conversion is monitored by ¹H NMR by analysis of dried samples in deuterated trifluoroacetic Acid. A conversion of 90.0% is reached in 6.9 hours. The conversion is 94.0% in 8.3 hours.

The reaction mixture is then filtered hot on sintered glass to remove the insoluble (residual oligomers). After cooling the filtrate down to 1-2°C, the adipic acid precipitates and can be recovered by filtration. The crude adipic acid is then purified by recrystallization.

The filtrates are then neutralised with sodium hydroxide 35% to release the diamine (hexamethylene diamine). The diamine is then recovered by distillation from the solution.

### Examples 2-6 and comparative example 7: hydrolysis of PA66 with HCl

With the same reactor and the same polyamide, the proportions of polyamide, HCl and water are modified. In all cases full dissolution of PA66 granules is observed in 65 to 40 min. A conversion ratio R of 90.0% can be obtained in all cases (see Table I).

**Table I**

| **Example** | **Ex 1** | **Ex 2** | **Ex 3** | **Ex 4** | **Ex 5** | **CEx 7** |
|---|---|---|---|---|---|---|
| PA66 (g) | 30.0 | 45.0 | 45.0 | 30.0 | 30.0 | 20.0 |
| HCl, 37% (g) | 78.4 | 43.1 | 49.0 | 52.2 | 65.3 | 21.8 |
| added water (g) | 0 | 0.0 | 0.0 | 0.0 | 0.0 | 80.0 |
| PA66 (wt.%) | 27.7 | 51.1 | 47.9 | 36.5 | 31.5 | 16.4 |
| HCl (wt.%) | 26.8 | 18.1 | 19.3 | 23.5 | 25.4 | 6.6 |
| H₂O (wt.%) | 45.6 | 30.8 | 32.8 | 40.0 | 43.2 | 77.0 |
| H/N | 3.00 | 1.10 | 1.25 | 2.00 | 2.50 | 1.25 |
| approx. dissolution time (min) visual) | 45 | 65 | 60 | 60 | 40 | incomplete dissolution |
| hrs of step b) to reach R=90% | 6.9 | 9.2 | 8.5 | 7.2 | 7.1 | 90.0% not reached |
| Productivity ratio R* (%) | 34.4 | 94.1 | 82.7 | / | / | / |

### Comparative example 7 (CEx 7): hydrolysis of PA66 with HCl

The reactor of example 1 is loaded with 20.0 g of polyamide 66 (0.177 mol amide functions), 21.8 g of HCl 37% (0.221 mol of proton, N/H = 1.25) and 80.0 g of distilled water.

The reaction mixture is stirred and heated to reflux in an oil bath at 120°C for 48 hours. In these conditions, [PA66] = 16.4%, [acid] = 6.6%, [H₂O] = 77.0%. The full dissolution of PA66 is never obtained and the hydrolysis remains incomplete.

### Examples 8-11 and comparative examples 12-13: hydrolysis of PA66 with HCl or H₂SO₄

In these examples, the same PA66 was hydrolysed in acidic conditions with the proportions indicated in Table II. The hydrolysis was performed at 100°C in sealed tubes. The content of all the tubes was analysed after 24 hours at 100°C.

**Table II**

| **Example** | **Ex 8** | **Ex 9** | **Ex 10*** | **Ex 11*** | **CEx 12** | **CEx 13** |
|---|---|---|---|---|---|---|
| PA66 (g) | 2.0 | 2.0 | 2.0 | 2.0 | 2.0 | 2.0 |
| Acid | HCl | HCl | H₂SO₄ | H₂SO₄ | H₂SO₄ | H₂SO₄ |
| acid (g) | 5.2 | 10.4 | 2.7 | 5.4 | 2.7 | 5.4 |
| water added (g) | 3.3 | 0.08 | 3.2 | 6.4 | 0.7 | 1.5 |
| PA66 (wt.%) | 19.0 | 16.0 | 25.4 | 14.5 | 36.8 | 22.6 |
| acid (wt.%) | 18.3 | 30.8 | 32.7 | 37.5 | 48.1 | 58.3 |
| H₂O (wt.%) | 62.7 | 53.2 | 41.9 | 48.0 | 15.1 | 19.0 |
| H / N | 2.99 | 1.24 | 2.97 | 5.96 | 3.01 | 5.95 |
| Degree of hydrolysis R (%) | 93.0 | 80.0 | 93.0 | 91.0 | 49.0 | 43.0 |
| Productivity ratio R* (%) | 21.8 | / | 31.7 | 15.4 | / | / |

| | | | | | | |
|---|---|---|---|---|---|---|
| Acid (Ac): HCl, 37 wt.% or H₂SO₄, 96 wt.% * not an example according to the invention | | | | | | |

### Example 14: hydrolysis of PA66 with HCl and removal of HCl

This example illustrates the hydrolysis of PA66 with HCl and recovery of the acid (Ac). The glass reactor of example 1 is loaded with 40.0 g of PA6,6 (0.363 mol of amide bonds) and 209.0 g of HCl 37% (2.128 mol, H/N = 6.0). The reaction mixture is stirred and heated to reflux for 24 h. The ¹H NMR analysis gives a conversion of 98.5% calculated on amine end groups and 98.1% calculated on acidic end groups.

A sample of the reaction mixture at the end of the reaction is diluted in a 50/50 weight mixture of water/trifluororethanol and potentiometric titration using 1.0N sodium hydroxide solution indicates that the reaction mixture comprises free HCl at a concentration of 5074 meq/kg (this corresponds to 1264 meq HCl in the final 249 g of reaction mixture).

The oil bath temperature is then raised to 130°C to eliminate HCl and water. 110 g of distillates are recovered. The potentiometric titration of the reaction mixture shows that the concentration of free HCl has decreased down to 425 meq/kg (this corresponds to 59 meq of HCl in the remaining 139 g of reaction mixture).

After this step, the neutralization of the recovered reaction mixture with NaOH generates thus only 25 g of NaCl (residual free HCl and HMD salt), compared to the initially expected 95 g (calculated from the amount of HCl titrated in the reaction mixture before distillation).

The removal of the excess acid HCl used for the hydrolysis is efficient and greatly reduces the amount of salt generated, thus reducing the environmental impact of the overall recycling process. The removal was performed at the laboratory level by distilling off HCl. On a larger scale, the acid would be removed by using different equipment.

R = 98.0% ; R* = 19.0%; α = 1.16 (= [59 meq HCl titrated + 363 meq of amine salt in the form -NH₃⁺Cl⁻] / 363 meq amide bond loaded). Without the distillation step to remove and recover the HCl, the alpha value would have been much higher.

### Examples 15-16: hydrolysis of MXD6 with HCl or H₂SO₄

This tests were performed in sealed tubes as in example 8. The hydrolysis was performed at 100°C in sealed tubes. The content of all the tubes was analysed after 24 hours at 100°C.

The results in Table III demonstrate that the method can be applied to recycled MXD6, a polyamide comprising aromatic moieties (polyamide prepared by the polycondensation of MXDA and adipic acid.

**Table III**

| **Example** | **Ex 15** | **Ex 16** |
|---|---|---|
| MXD6 (g) | 2.0 | 2.0 |
| Acid | HCl | H₂SO₄ |
| acid (g) | 4.8 | 2.4 |
| water added (g) | 3.7 | 3.3 |
| MXD6 (wt.%) | 19.2 | 26.0 |
| acid (wt.%) | 17.0 | 30.2 |
| H₂O (wt.%) | 63.9 | 43.8 |
| H / N | 2.99 | 2.91 |
| Degree of hydrolysis R (%) | 98.0 | 99.0 |
| Productivity ratio R* (%) | 23.3 | 34.8 |

| | | |
|---|---|---|
| Acid (Ac): HCl, 37 wt.% or H₂SO₄, 96 wt.% | | |

### Examples 17-18: hydrolysis of a copolyamide 66/6T (35/65)

The reagents listed in the table below are loaded in a 300 ml glass reactor equipped with a reflux condenser and a mechanical stirrer. The reaction mixture is immersed in an oil bath at 120°C and kept at reflux for 24 hours. During the whole time, the reaction mixture remains turbid. The hydrolysis conversion is monitored by ¹H NMR analysis of dried samples in deuterated trifluoroacetic acid.

**Table IV**

| **Example** | **Ex 16** | **Ex 17** | **Ex 18** |
|---|---|---|---|
| 66/6T 35/65 (g) | 40.0 | 40.0 | 30. |
| Acid | HCl | HCl | H₂SO₄ |
| acid (g) | 105 | 209 | 77 |
| water added (g) | 0 | 1.7 | 47.0 |
| 66/6T 35/65 (wt.%) | 28 | 16 | 20 |
| acid (wt.%) | 27 | 31 | 49 |
| H₂O (wt.%) | 45 | 53 | 21 |
| H/N | 3 | 6 | 6 |
| Degree of hydrolysis R (%) | 57% | 64% | 80% |

| | | | |
|---|---|---|---|
| Acid (Ac): HCl, 37 wt.% or H₂SO₄, 98 wt.% | | | |

### Example 19: acidic hydrolysis of a polyamide 6.10 with HCl

A 300 ml glass reactor equipped with a reflux condenser and a mechanical stirrer is loaded with 40.0 g of recycled pellets (3-5 mm) of unfilled polyamide 6.10 (100% of PA, 0.283 mol of amide bonds) and 83.75 g of hydrochloric acid 37 wt.% aqueous solution (0.850 mol). The reaction mixture is immersed in an oil bath at 120°C and kept at reflux for 24 hours. During the whole time, the reaction mixture remains turbid. Samples are taken regularly and dried in a vacuum oven overnight. The hydrolysis

The conversion is monitored by ¹H NMR by analysis of dried samples in deuterated trifluoroacetic Acid. The hydrolysis conversion is 87% after 12 hours, and 97% after 24 hours.

### Example 20: acidic hydrolysis of PA6.10 with the use of ethanol

A 300 ml glass reactor equipped with a reflux condenser and a mechanical stirrer is loaded with 30.0 g of recycled pellets (3-5 mm) of unfilled polyamide 6.10 (100% of PA, 0.212 mol of amide bonds), 62.81 g of hydrochloric acid (37 wt.% aqueous solution; 0.637 mol), 10.47 g of deionized water and 73.25 g of absolute ethanol. The reaction mixture is immersed in an oil bath at 105°C and kept at reflux for 48 hours. During the whole time, the reaction mixture remains turbid. The hydrolysis conversion is determined by ¹H NMR on dried samples in deuterated trifluoroacetic Acid. The hydrolysis conversion is 84% after 24 hours. 40% of the acid end groups are under the acid form and 60% under the ethyl ester form.

## Claims

1. Method of recovery of the monomers from a polyamide (PA) of the AABB type comprising the following steps:
- a) a product (P) comprising the polyamide (PA) is optionally comminuted;
- b) an aqueous medium comprising water, the product (P) and an acid (Ac) is heated to induce the dissolution and the hydrolysis of the polyamide (PA);
- c) a stream (S) comprising the diacid (A) and the diamine (B) in the form of a salt with the acid (Ac), recovered at the end of step b) is further processed in order to either separate and recover the diacid (A) and the diamine (B) (embodiment E1) or collect the salt formed by the diacid (A) and the diamine (B) (embodiment E2);
wherein the acid (Ac) is selected in the group consisting of HCl, HBr, methane sulfonic acid and combinations of two or more of these acids;
wherein step b) is performed with the following conditions:
(i) the initial proportions in the aqueous medium are the following:
- proportion of polyamide (PA): at least 15.0 wt.%;
- proportion of water: between 20.0 and 70.0 wt.%;
- the remainder as acid (Ac) with the condition that the proportion of acid (Ac) is at least 2.0 wt.%, preferably at least 2.5 wt.%;
these proportions being based on the total weight of polyamide (PA), water and acid (Ac);
- the molar ratio (H / N) representing the amount of H from the acid (Ac) over the amount of N from the amide bonds of the polyamide (PA) between 1.1 and 7.0;
(ii) the temperature is between 80°C and 150°C;
wherein after step b), the method comprises a total or partial removal of the unreacted acid (Ac) from the reaction mixture;
wherein:
- according to embodiment (E1), step c) comprises a separation substep c1), wherein the diacid (A) and the salt of the diamine (B) are separated and a neutralisation substep c2), wherein the salt of diamine (B) separated from the diacid (A) is neutralised with at least one inorganic base of formula XOH, X being Li, Na, K or a combination of two or more of these cations to release the diamine (B);
- according to embodiment (E2), step c) comprises a neutralisation substep c2) in which the salt of diamine (B) is neutralised with an inorganic base of formula XOH to release the diamine (B);
and wherein the α ratio is less than or equal to 2.0, this ratio being defined as the molar ratio = [salt of the acid (Ac) with XOH released by the method] / [amide bonds of the polyamide (PA)].

2. Method according to claim 1, wherein the polyamide (PA) of the AABB type is prepared by polycondensation of:
- at least one diacid (A) selected in the group of C₃-C₁₈ aliphatic diacids, isophthalic acid and terephthalic acid; and
- at least one diamine (B) selected in the group consisting of C₂-C₁₈ aliphatic diamines, C₄-C₁₈ cycloaliphatic diamines and diamines of formula (IV): 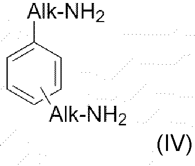 wherein Alk is a C₁-C₆ linear or branched alkylene group.

3. Method according to claim 1 or claim 2, wherein the polyamide (PA) is selected in the group consisting of polyamide 6.6, polyamide 6.9, polyamide 6.10, polyamide 6.12, polyamide 10.10, polyamide 4.6, polyamide 4.9, polyamide 4.10, polyamide 12.12, polyamide 10.12, polyamide MXD6, polyamide MXD6/PXD6, polyamide MXD6/MXDI, polyamide 6T/66, polyamide 6T/6I/66, PA 6T/6I and mixtures thereof.

4. Method according to any one of the preceding claims wherein the initial proportions in the liquid medium are the following:
- proportion of polyamide (PA): between 15.0 and 55.0 wt.%;
- proportion of water: between 20.0 and 70.0 wt.%;
- proportion of acid (Ac): between 10.0 and 55.0 wt.%;
- H/N between 2.0 and 7.0.
or the following:
- proportion of polyamide (PA): between 15.0 and 35.0 wt.%;
- proportion of water: between 20.0 and 55.0 wt.%;
- proportion of acid (Ac): between 12.0 and 50.0 wt.%;
- H/N between 2.5 and 3.5.
or the following:
- proportion of polyamide (PA): between 15.0 and 30.0 wt.%;
- proportion of water: between 30.0 and 45.0 wt.%;
- proportion of acid (Ac): between 12.0 and 50.0 wt.%;
- H/N between 2.5 and 3.5.

5. Method according to any one of the preceding claims wherein the proportion of water in the aqueous medium is between 30.0 wt.% and 70.0 wt.% or between 35.0 wt.% and 65.0 wt.%.

6. Method according to any one of the preceding claims wherein the initial molar ratio (H / N) is at least 2.0 or between 2.5 and 7.0.

7. Method according to any one of the preceding claims, wherein the temperature at which step b) is performed is between 100°C and 130°C.

8. Method according to any one of the preceding claims, wherein the degree of hydrolysis R achieved at the end of step b) is preferably at least 90.0 mol%, preferably at least 95.0 mol%, this ratio corresponding to the degree of conversion of the hydrolysis of the polyamide (PA).

9. Method according to claim 8, wherein a degree of hydrolysis R of at least 90.0 mol%, preferably at least 95.0 mol%, is achieved for a duration of step b) of less than 9.0 hours, preferably less than 8.0 hours.

10. Method according to any one of the preceding claims wherein a productivity ratio R* of at least 20.0%, preferably at least 25.0%, preferably at least 30.0%, is achieved for a duration of step b), the productivity ratio being defined for a degree of hydrolysis R of at least 90.0% as: initial proportion in wt.% of polyamide (PA) x R (%) / (initial proportion in wt.% of water + initial proportion in wt.% of acid) x 100.

11. Method according to any one of the preceding claims, wherein the solid materials that may be present in the stream (S) are partly or totally removed by filtration of stream (S) prior to step c).

12. Method according to any one of the preceding claims wherein α is less than or equal to 1.5 (≤ 1.5), preferably less than or equal to 1.4 (≤ 1.4), preferably less than or equal to 1.3 (≤ 1.3), preferably less than or equal to 1.2 (≤ 1.2), preferably less than or equal to 1.1 (≤ 1.1).

13. Method according to any one of the preceding claims, wherein the duration of the hydrolysis from a degree of hydrolysis of R=0 to R=90.0% is between 6.0 and 10.0 hours.

14. Method according to any one of the preceding claims, wherein the total or partial removal of the unreacted acid (Ac) from the reaction mixture is performed by heating and/or applying vacuum to the reaction mixture.

## Patentansprüche

1. Verfahren zur Rückgewinnung der Monomere aus einem Polyamid (PA) des AABB-Typs, umfassend die folgenden Schritte:
- a) ein Produkt (P), das das Polyamid (PA) umfasst, wird gegebenenfalls zerkleinert;
- b) ein wässriges Medium, das Wasser, das Produkt (P) und eine Säure (Ac) umfasst, wird erhitzt, um die Auflösung und die Hydrolyse des Polyamids (PA) herbeizuführen;
- c) ein Strom (S), der die Disäure (A) und das Diamin (B) in Form eines Salzes mit der Säure (Ac) umfasst, der am Ende von Schritt b) gewonnen wird, wird weiterverarbeitet, um entweder die Disäure (A) und das Diamin (B) zu trennen und zurückzugewinnen (Ausführungsform E1) oder das durch die Disäure (A) und das Diamin (B) gebildete Salz zu sammeln (Ausführungsform E2);
wobei die Säure (Ac) aus der Gruppe ausgewählt ist, die aus HCl, HBr, Methansulfonsäure und Kombinationen von zwei oder mehr dieser Säuren besteht;
wobei Schritt b) unter den folgenden Bedingungen durchgeführt wird:
(i) die anfänglichen Anteile in dem wässrigen Medium sind wie folgt:
- Anteil an Polyamid (PA): mindestens 15,0 Gew.-%;
- Anteil an Wasser: zwischen 20,0 und 70,0 Gew.-%;
- der Rest als Säure (Ac) unter der Bedingung, dass der Anteil an Säure (Ac) mindestens 2,0 Gew.-%, vorzugsweise mindestens 2,5 Gew.-%, beträgt;
wobei diese Anteile auf dem Gesamtgewicht von Polyamid (PA), Wasser und Säure (Ac) basieren;
- das Molverhältnis (H / N), das die Menge an H aus der Säure (Ac) gegenüber der Menge an N aus den Amidbindungen des Polyamids (PA) darstellt, liegt zwischen 1,1 und 7,0;
(ii) die Temperatur liegt zwischen 80°C und 150°C;
wobei das Verfahren nach Schritt b) eine vollständige oder teilweise Entfernung der nicht umgesetzten Säure (Ac) aus dem Reaktionsgemisch umfasst;
wobei:
- gemäß Ausführungsform (E1) Schritt c) einen Trennteilschritt c1) umfasst, wobei die Disäure (A) und das Salz des Diamins (B) getrennt werden, und einen Neutralisationsteilschritt c2), wobei das von der Disäure (A) getrennte Salz des Diamins (B) mit mindestens einer anorganischen Base der Formel XOH neutralisiert wird, wobei X Li, Na, K oder eine Kombination von zwei oder mehr dieser Kationen ist, um das Diamin (B) freizusetzen;
- gemäß Ausführungsform (E2) Schritt c) einen Neutralisationsteilschritt c2) umfasst, in dem das Salz des Diamins (B) mit einer anorganischen Base der Formel XOH neutralisiert wird, um das Diamin (B) freizusetzen;
und wobei das α-Verhältnis kleiner oder gleich 2,0 ist, wobei dieses Verhältnis definiert ist als das Molverhältnis = [durch das Verfahren freigesetztes Salz der Säure (Ac) mit XOH] / [Amidbindungen des Polyamids (PA)].

2. Verfahren nach Anspruch 1, wobei das Polyamid (PA) des AABB-Typs durch Polykondensation hergestellt wird aus:
- mindestens einer Disäure (A), ausgewählt aus der Gruppe der C₃-C₁₈ aliphatischen Disäuren, Isophthalsäure und Terephthalsäure; und
- mindestens einem Diamin (B), ausgewählt aus der Gruppe bestehend aus C₂-C₁₈ aliphatischen Diaminen, C₄-C₁₈ cycloaliphatische Diaminen und Diaminen der Formel (IV): 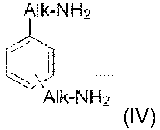
- wobei Alk eine lineare oder verzweigte C₁-C₆ Alkylengruppe ist.

3. Verfahren nach Anspruch 1 oder Anspruch 2, wobei das Polyamid (PA) aus der Gruppe ausgewählt ist, bestehend aus Polyamid 6.6, Polyamid 6.9, Polyamid 6.10, Polyamid 6.12, Polyamid 10.10, Polyamid 4.6, Polyamid 4.9, Polyamid 4.10, Polyamid 12.12, Polyamid 10.12, Polyamid MXD6, Polyamid MXD6/PXD6, Polyamid MXD6/MXDI, Polyamid 6T/66, Polyamid 6T/6I/66, PA 6T/6I und Mischungen davon.

4. Verfahren nach einem der vorhergehenden Ansprüche, wobei die anfänglichen Anteile in dem flüssigen Medium wie folgt sind:
- Anteil an Polyamid (PA): zwischen 15,0 und 55,0 Gew.-%;
- Anteil an Wasser: zwischen 20,0 und 70,0 Gew.-%;
- Anteil an Säure (Ac): zwischen 10,0 und 55,0 Gew.-%;
- H/N zwischen 2,0 und 7,0.
oder wie folgt:
- Anteil an Polyamid (PA): zwischen 15,0 und 35,0 Gew.-%;
- Anteil an Wasser: zwischen 20,0 und 55,0 Gew.-%;
- Anteil an Säure (Ac): zwischen 12,0 und 50,0 Gew.-%;
- H/N zwischen 2,5 und 3,5.
oder wie folgt:
- Anteil an Polyamid (PA): zwischen 15,0 und 30,0 Gew.-%;
- Anteil an Wasser: zwischen 30,0 und 45,0 Gew.-%;
- Anteil an Säure (Ac): zwischen 12,0 und 50,0 Gew.-%;
- H/N zwischen 2,5 und 3,5.

5. Verfahren nach einem der vorhergehenden Ansprüche, wobei der Anteil an Wasser in dem wässrigen Medium zwischen 30,0 Gew.-% und 70,0 Gew.-% oder zwischen 35,0 Gew.-% und 65,0 Gew.-% liegt.

6. Verfahren nach einem der vorhergehenden Ansprüche, wobei das anfängliche Molverhältnis (H / N) mindestens 2,0 beträgt oder zwischen 2,5 und 7,0 liegt.

7. Verfahren nach einem der vorhergehenden Ansprüche, wobei die Temperatur, bei der Schritt b) durchgeführt wird, zwischen 100°C und 130°C liegt.

8. Verfahren nach einem der vorhergehenden Ansprüche, wobei der am Ende von Schritt b) erreichte Hydrolysegrad R vorzugsweise mindestens 90,0 Mol-%, vorzugsweise mindestens 95,0 Mol-%, beträgt, wobei dieses Verhältnis dem Umsetzungsgrad der Hydrolyse des Polyamids (PA) entspricht.

9. Verfahren nach Anspruch 8, wobei ein Hydrolysegrad R von mindestens 90,0 Mol-%, vorzugsweise mindestens 95,0 Mol-%, für eine Dauer von Schritt b) von weniger als 9,0 Stunden, vorzugsweise weniger als 8,0 Stunden, erreicht wird.

10. Verfahren nach einem der vorhergehenden Ansprüche, wobei ein Produktivitätsverhältnis R* von mindestens 20,0%, vorzugsweise mindestens 25,0%, vorzugsweise mindestens 30,0%, für eine Dauer von Schritt b) erreicht wird, wobei das Produktivitätsverhältnis für einen Hydrolysegrad R von mindestens 90,0% definiert ist als: anfänglicher Anteil an Polyamid (PA) in Gew.-% × R (%) / (anfänglicher Anteil an Wasser in Gew.-% + anfänglicher Anteil an Säure in Gew.-%) × 100.

11. Verfahren nach einem der vorhergehenden Ansprüche, wobei die festen Materialien, die in dem Strom (S) vorhanden sein können, vor Schritt c) durch Filtration des Stroms (S) teilweise oder vollständig entfernt werden.

12. Verfahren nach einem der vorhergehenden Ansprüche, wobei α kleiner oder gleich 1,5 (≤ 1,5), vorzugsweise kleiner oder gleich 1,4 (≤ 1,4), vorzugsweise kleiner oder gleich 1,3 (≤ 1,3), vorzugsweise kleiner oder gleich 1,2 (≤ 1,2), vorzugsweise kleiner oder gleich 1,1 (≤ 1,1) ist.

13. Verfahren nach einem der vorhergehenden Ansprüche, wobei die Dauer der Hydrolyse von einem Hydrolysegrad von R=0 bis R=90,0% zwischen 6,0 und 10,0 Stunden liegt.

14. Verfahren nach einem der vorhergehenden Ansprüche, wobei die vollständige oder teilweise Entfernung der nicht umgesetzten Säure (Ac) aus dem Reaktionsgemisch durch Erhitzen und/oder Anlegen von Vakuum an das Reaktionsgemisch durchgeführt wird.

## Revendications

1. Procédé de récupération des monomères à partir d'un polyamide (PA) de type AABB comprenant les étapes suivantes :
- a) un produit (P) comprenant le polyamide (PA) est éventuellement broyé ;
- b) un milieu aqueux comprenant de l'eau, le produit (P) et un acide (Ac) est chauffé pour provoquer la dissolution et l'hydrolyse du polyamide (PA) ;
- c) un flux (S) comprenant le diacide (A) et la diamine (B) sous la forme d'un sel avec l'acide (Ac), récupéré à la fin de l'étape b), est soumis à un traitement supplémentaire afin soit de séparer et récupérer le diacide (A) et la diamine (B) (mode de réalisation E1), soit de recueillir le sel formé par le diacide (A) et la diamine (B) (mode de réalisation E2) ;
dans lequel l'acide (Ac) est choisi dans le groupe constitué de HCl, HBr, de l'acide méthanesulfonique et de combinaisons de deux ou plusieurs de ces acides ;
dans lequel l'étape b) est réalisée dans les conditions suivantes :
(i) les proportions initiales dans le milieu aqueux sont les suivantes :
- proportion de polyamide (PA) : au moins 15,0 % en poids ;
- proportion d'eau : entre 20,0 et 70,0 % en poids ;
- le reste étant constitué de l'acide (Ac), à condition que la proportion d'acide (Ac) soit d'au moins 2,0 % en poids, de préférence d'au moins 2,5 % en poids ;
ces proportions étant fondées sur le poids total de polyamide (PA), d'eau et d'acide (Ac) ;
- le rapport molaire (H / N) représentant la quantité de H provenant de l'acide (Ac) par rapport à la quantité de N provenant des liaisons amide du polyamide (PA) étant compris entre 1,1 et 7,0 ;
(ii) la température est comprise entre 80°C et 150°C ;
dans lequel, après l'étape b), le procédé comprend une élimination totale ou partielle de l'acide (Ac) n'ayant pas réagi du mélange réactionnel ;
dans lequel :
- selon le mode de réalisation (E1), l'étape c) comprend une sous-étape de séparation c1), dans laquelle le diacide (A) et le sel de la diamine (B) sont séparés, et une sous-étape de neutralisation c2), dans laquelle le sel de diamine (B) séparé du diacide (A) est neutralisé avec au moins une base inorganique de formule XOH, X étant Li, Na, K ou une combinaison de deux ou plusieurs de ces cations pour libérer la diamine (B) ;
- selon le mode de réalisation (E2), l'étape c) comprend une sous-étape de neutralisation c2) dans laquelle le sel de diamine (B) est neutralisé avec une base inorganique de formule XOH pour libérer la diamine (B) ;
et dans lequel le rapport α est inférieur ou égal à 2,0, ce rapport étant défini comme le rapport molaire = [sel de l'acide (Ac) avec XOH libéré par le procédé] / [liaisons amide du polyamide (PA)].

2. Procédé selon la revendication 1, dans lequel le polyamide (PA) de type AABB est préparé par polycondensation de :
- au moins un diacide (A) choisi dans le groupe des diacides aliphatiques en C₃-C₁₈, de l'acide isophtalique et de l'acide téréphtalique ; et
- au moins une diamine (B) choisie dans le groupe constitué des diamines aliphatiques en C₂-C₁₈, des diamines cycloaliphatiques en C₄-C₁₈ et des diamines de formule (IV) : 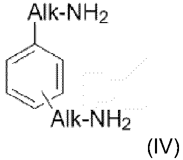
- dans laquelle Alk est un groupe alkylène linéaire ou ramifié en C₁-C₆.

3. Procédé selon la revendication 1 ou la revendication 2, dans lequel le polyamide (PA) est choisi dans le groupe constitué du polyamide 6.6, du polyamide 6.9, du polyamide 6.10, du polyamide 6.12, du polyamide 10.10, du polyamide 4.6, du polyamide 4.9, du polyamide 4.10, du polyamide 12.12, du polyamide 10.12, du polyamide MXD6, du polyamide MXD6/PXD6, du polyamide MXD6/MXDI, du polyamide 6T/66, du polyamide 6T/6I/66, du PA 6T/6I et de leurs mélanges.

4. Procédé selon l'une quelconque des revendications précédentes, dans lequel les proportions initiales dans le milieu liquide sont les suivantes :
- proportion de polyamide (PA) : entre 15,0 et 55,0 % en poids ;
- proportion d'eau : entre 20,0 et 70,0 % en poids ;
- proportion d'acide (Ac) : entre 10,0 et 55,0 % en poids ;
- H/N entre 2,0 et 7,0.
ou les suivantes :
- proportion de polyamide (PA) : entre 15,0 et 35,0 % en poids ;
- proportion d'eau : entre 20,0 et 55,0 % en poids ;
- proportion d'acide (Ac) : entre 12,0 et 50,0 % en poids ;
- H/N entre 2,5 et 3,5.
ou les suivantes :
- proportion de polyamide (PA) : entre 15,0 et 30,0 % en poids ;
- proportion d'eau : entre 30,0 et 45,0 % en poids ;
- proportion d'acide (Ac) : entre 12,0 et 50,0 % en poids ;
- H/N entre 2,5 et 3,5.

5. Procédé selon l'une quelconque des revendications précédentes, dans lequel la proportion d'eau dans le milieu aqueux est comprise entre 30,0 % en poids et 70,0 % en poids ou entre 35,0 % en poids et 65,0 % en poids.

6. Procédé selon l'une quelconque des revendications précédentes, dans lequel le rapport molaire initial (H / N) est d'au moins 2,0 ou compris entre 2,5 et 7,0.

7. Procédé selon l'une quelconque des revendications précédentes, dans lequel la température à laquelle l'étape b) est réalisée est comprise entre 100°C et 130°C.

8. Procédé selon l'une quelconque des revendications précédentes, dans lequel le degré d'hydrolyse R atteint à la fin de l'étape b) est de préférence d'au moins 90,0 % en moles, de préférence d'au moins 95,0 % en moles, ce rapport correspondant au degré de conversion de l'hydrolyse du polyamide (PA).

9. Procédé selon la revendication 8, dans lequel un degré d'hydrolyse R d'au moins 90,0 % en moles, de préférence d'au moins 95,0 % en moles, est atteint pour une durée de l'étape b) inférieure à 9,0 heures, de préférence inférieure à 8,0 heures.

10. Procédé selon l'une quelconque des revendications précédentes, dans lequel un rapport de productivité R* d'au moins 20,0 %, de préférence d'au moins 25,0 %, de préférence d'au moins 30,0 %, est atteint pour une durée de l'étape b), le rapport de productivité étant défini pour un degré d'hydrolyse R d'au moins 90,0 % comme suit : proportion initiale en % en poids de polyamide (PA) x R (%) / (proportion initiale en % en poids d'eau + proportion initiale en % en poids d'acide) x 100.

11. Procédé selon l'une quelconque des revendications précédentes, dans lequel les matières solides pouvant être présentes dans le flux (S) sont partiellement ou totalement éliminées par filtration du flux (S) avant l'étape c).

12. Procédé selon l'une quelconque des revendications précédentes, dans lequel α est inférieur ou égal à 1,5 (≤ 1,5), de préférence inférieur ou égal à 1,4 (≤ 1,4), de préférence inférieur ou égal à 1,3 (≤ 1,3), de préférence inférieur ou égal à 1,2 (≤ 1,2), de préférence inférieur ou égal à 1,1 (≤ 1,1).

13. Procédé selon l'une quelconque des revendications précédentes, dans lequel la durée de l'hydrolyse d'un degré d'hydrolyse de R=0 à R=90,0 % est comprise entre 6,0 et 10,0 heures.

14. Procédé selon l'une quelconque des revendications précédentes, dans lequel l'élimination totale ou partielle de l'acide (Ac) n'ayant pas réagi du mélange réactionnel est réalisée par chauffage et/ou application d'un vide au mélange réactionnel.
